# EUROPEAN PATENT APPLICATION

(11) **EP 1 350 528 A1**
(43) Date of publication of application: **08.10.2003**
(21) Application number: 03002864.1
(22) Date of filing: 08.02.2003
(51) Int. Cl.: A61M 5/32, A61B 19/02, B65D 85/24

(54) **Needle-disposable insert for sharps disposal**

(30) Priority: 28.03.2002 US 368581 P; 18.10.2002 US 274673
(71) Applicant: Becton, Dickinson and Company, Franklin Lakes, New Jersey 07417-1880 (US)
(72) Inventor: Crawford, Jamieson William Maclean, New York, New York 10025 (US)
(74) Representative: von Kreisler, Alek, Dipl.-Chem.

(57) **Abstract**

An infectious waste container system provides a sharps disposal container with an insert for automatically releasing a sharp implement from a holder and automatically resetting the holder.

## Description

### 1. Field of the Invention

The present invention relates to an infectious waste container system for fluid collection needles. More particularly, the invention relates to an improvement in the disposal of needles from a needle holder into a sharps needle disposal container.

### 2. Background of the Invention

Fluid collection needles are used for sampling fluid, such as blood from a patient for tests or blood sedimentation. The needles usually are affixed to a needle holder by screw engagement. A used needle is separated from a holder for discard by covering the used needle with a protector and disengaging the male screw on the hub of the needle from the female screw of the needle holder to separate the needle, and the protector from the holder. The removed needle, covered by the protector, then is put into a suitable container.

In this process however, there is a risk of an accidental needle stick with a finger or a palm of the hand at the time of covering the blood collection needle with the protector. There are obvious biohazards associated with such a procedure for the operator, i.e., infection with viruses, such as HIV or hepatitis in the worse case.

To solve this problem, devices have been developed, including various needle holders, which are designed to allow the operator to remove the used blood collection needle without handling it with fingers. These needle holders specifically are designed to be easily operable and to be used in combination with a needle having a specific configuration including a concave or convex portion. However, due to the specific configuration, these needle holders cannot be used in combination with conventional screw mount type needles.

To solve the problem of assembling the needle to the needle holder, more sophisticated needle holders have been developed including an easily operable needle holder. The easily operable needle holder includes a cylindrical holder body with first and second ends. The cylindrical holder body has a needle fixing means at the first end of the cylinder. The needle fixing means includes a fixed member and first and second sliding members that are slidable along a wall at the first end of the cylindrical body. The fixed member is configured to grip one circumferential half of a needle hub, and the first sliding member is configured to grip the opposed circumferential half of the needle hub. The first sliding member is movable between a closed position where the needle hub is gripped between the fixed member and the first sliding member and an open position where the needle hub can pass between the fixed member and the first sliding member. The second sliding member is effectively an actuator that can move the first sliding member from the closed position to the open position. This needle holder makes it possible to fix the needle releasably to the holder and may be used in combination with screw-mount type blood collection needles.

The needle can be removed from the above-described holder by exerting digital pressure on the second sliding member to slide the first sliding member away from the fixed member. This disengages the needle from the holder, and the needle falls gravitationally into a suitable sharps container located just below the needle holder. To facilitate the depositing of the needles from the holder into such disposable sharps containers, inserts fitted into the container have been developed to activate the release mechanism on the holder for disengaging the needle from the holder. Such an insert typically includes a release mechanism which contacts the first sliding member, thereby sliding the first sliding member toward the second sliding member, and disengaging the needle from the holder.

There are a number of drawbacks to such a disposal system however. While the procedure for disengaging the needle from the needle holder has been made somewhat more efficient, there remains a need to restart the needle holder. Restarting a needle holder occurs by pushing the second sliding member toward the first sliding member so that the needle holder is in a position for grasping a new needle.

### 3. Summary of the Invention

The present invention provides an improved needle disposal insert for a disposal container. The needle disposal insert includes a cylindrical member having a cylindrical wall including atop end and a bottom end. First and second recesses are diametrically opposed on the cylindrical wall. The first recess includes a notched portion for disengaging a needle from a needle holder, and the second recess includes an indented portion including a restart means for returning the needle holder to a closed position. The restart means in the second recess may comprise a sloped wall sloping radially outwardly from the top end of the insert for engaging a restart mechanism on a needle holder.

The present invention also provides an infectious waste container system that includes a disposal container, a needle holder, and a needle disposal insert mounted to or formed with the disposal container for interaction with the needle holder. The needle disposal insert comprises a cylindrical member having a cylindrical wall with top and bottom ends and having first and second diametrically opposed recess on the cylindrical wall. The first recess includes a notched portion for disengaging a needle from a needle collection holder. The second recess includes an indented portion with a restart means for restarting the needle holder to a closed position. The restart means in the second recess is a sloped wall sloping radially out from the top end of the insert and engages a restart mechanism on a needle holder.

### 4. Brief Description of the Drawings

FIG. 1 is a longitudinal cross-sectional view of a needle holder and needle assembly.

FIG. 2 is a longitudinal cross-sectional view of the needle holder in FIG. 1 after release of the needle assembly.

FIG 3. is a perspective view of a needle disposal insert in accordance with the invention.

FIG. 4 is a top plan view of the needle disposal insert.

FIG. 5 is a front elevational view of the needle disposal insert.

FIG. 6 is a side elevational view of the needle disposal insert.

FIG. 7 is a rear elevational view of the needle disposal insert.

FIG. 8 is a bottom plan view of the needle disposal insert.

FIG. 9 is a cross-sectional view taken along line 9-9 in FIG. 4.

FIG. 10 is a perspective view of a disposal container that includes the disposal insert of FIGS. 3-9.

FIG. 11 is a longitudinal cross-sectional view of the needle holder and needle assembly of FIG. 1 during an initial stage of insertion into the needle disposal insert of FIGS. 3-9.

FIG. 12 is a cross-sectional view similar to FIG. 11, but showing the needle holder inserted sufficiently into the needle disposal insert.

FIG. 13 is a cross-sectional view similar to FIG. 11, but showing an alternate needle disposal insert.

FIG. 14 is a cross-sectional view similar to FIG. 12, but showing the alternate needle disposal insert.

### 5. Detailed Description of the Invention

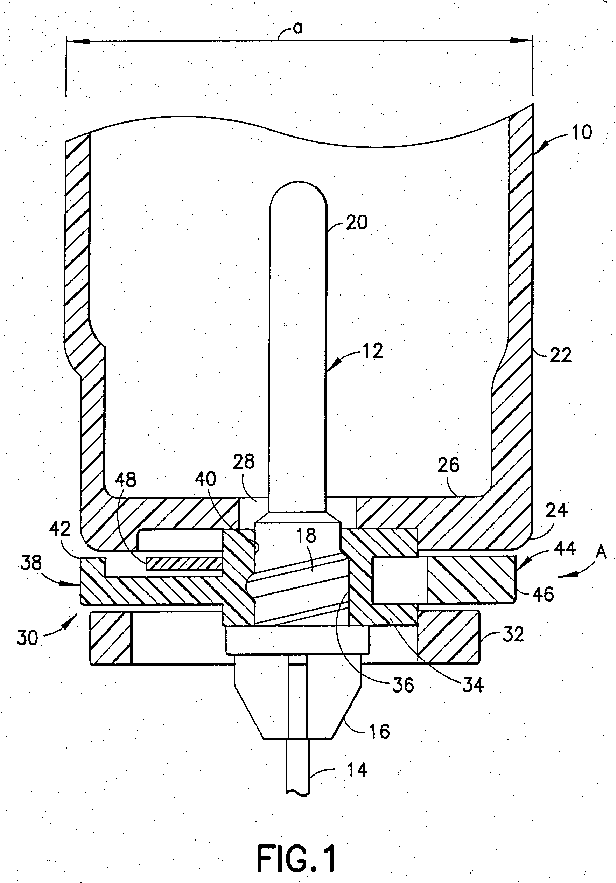

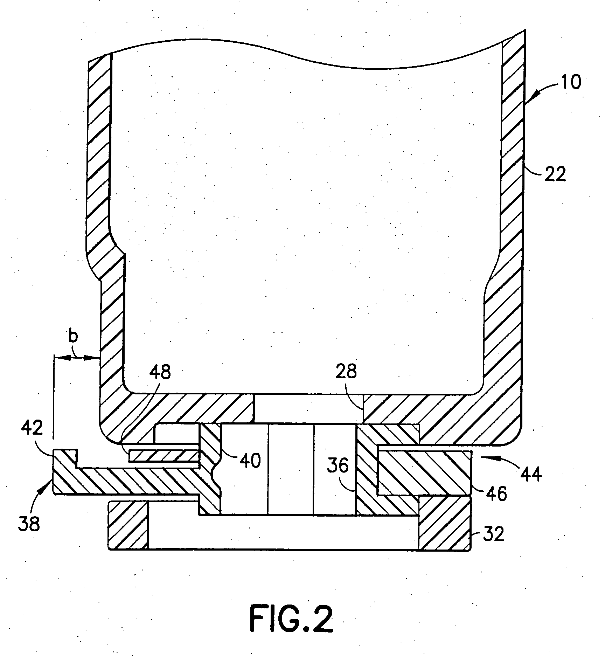

The needle disposal insert of the subject invention is used with a known needle holder **10** and a known needle assembly **12,** as shown in FIGS. 1 and 2. Needle assembly **12** includes a needle cannula **14** with an intravenous end facing downwardly in FIG. 1 and a non-patient end facing upwardly in FIG. 1. Needle cannula **14** is securely and permanently mounted in a plastic hub **16.** Hub **16** includes an array of external threads **18** that extends from an intermediate location on hub **16** toward the non-patient end of needle cannula **14.** An elastomeric sheath **20** covers the non-patient end of needle cannula **14** and functions as a valve to permit multiple samples of a bodily fluid to be collected.

Needle holder **10** includes a tubular sidewall **22** with a widely open proximal end (not shown) and a distal end **24**. An end wall **26** extends transversely across distal end **24** of tubular sidewall **22** and includes a central aperture **28.** Aperture **28** defines a cross-sectional dimension that exceeds the maximum cross-sectional dimension of needle assembly **12**.

A needle gripping assembly **30** is secured to end wall **26** of holder **10**. Needle gripping assembly **30** includes a housing **32** and a fixed jaw **34**, both of which are substantially immovably mounted to end wall **26.** Fixed jaw **34** includes a semi-cylindrical concave inner surface **36** that is concentric with aperture **28** in end wall **26** and that is dimensioned to engage the threaded portion of hub **16.** Needle gripping assembly **30** further includes a movable jaw **38** that is movable toward and away from fixed jaw **34.** Movable jaw **38** is characterized by a generally semi-cylindrical concave surface **40** that is configured to engage the threaded portion of hub **16** when movable jaw **38** is moved toward fixed jaw **34,** as shown in FIG. 1. Movable jaw 38 further includes a flange **42** spaced radially outwardly from concave gripping surface **40**. An actuating member 44 that is movable parallel to movable jaw **38**, and has an externally accessible first actuating surface **46** that is diametrically opposite concave gripping surface **40** of the movable jaw **38.** Actuating member **44** further includes a second actuating surface **48** that faces flange **42** of movable jaw **38.**

As shown most clearly in FIG. 1, sidewall **22** of needle holder **10** defines an outside diameter "a" at locations near end wall **26**. The outside cross-sectional dimension between the first actuating surface **46** of actuator **40** and flange **42** on movable jaw **38** is only slightly less than outside diameter "a". As shown in FIG. 2, the outside surface of flange **42** of movable jaw **38** is offset from the outer surface of tubular sidewall **22** on needle holder **10** by a distance "b" when movable jaw **38** is in the open position.

FIG. 1 depicts a condition where movable jaw **38** is moved toward fixed jaw **34.** In this position, threads **18** of hub **16** can be secured between concave gripping surfaces **36** and **40** for holding needle assembly **12** in needle holder **10.** In this position, the non-patient end of needle assembly **12** projects into needle holder **10**. Radially inward forces can be exerted on first actuating surface **46** of actuator **44** to move actuator **44** in the direction of arrow A in FIG. 1. This movement will cause actuator **44** to move movable jaw **38** away from fixed jaw **34** and into the condition shown in FIG. 2. In this disposition, the cross-section defined by semi-cylindrical concave gripping surfaces **36** and **40** exceeds the cross-sectional dimensions of threaded portion of needle hub **16.** As a result, needle assembly **16** will fall gravitationally from needle holder **10.** Needle holder **10** can be reset for accommodating a new needle assembly by exerting radially inward forces on flange **42** of movable jaw **38**. These forces urge movable jaw **38** into actuator **44**, and hence reset both movable jaw **38** and actuator **44** into a position for threadedly accommodating a new needle assembly **12.**

Some needle holders as described above and illustrated in FIGS. 1 and 2 are intended to be actuated and reset by a thumb or forefinger. However, as explained above, there are advantages to minimizing manual activity required near the needle assembly. Other needle holders are used with an actuating ring. The actuator ring typically is mounted in an opening in a sharps disposal container. The needle holder and needle assembly are urged into the actuator ring. The actuator ring is configured to urge the actuator in the direction of arrow A for moving a movable jaw and releasing the needle cannula. These designs, however, require the user to manually reset the movable jaw. The manual resetting of the movable jaw is an inconvenience, and if not performed properly could prevent correct engagement of the next needle assembly.

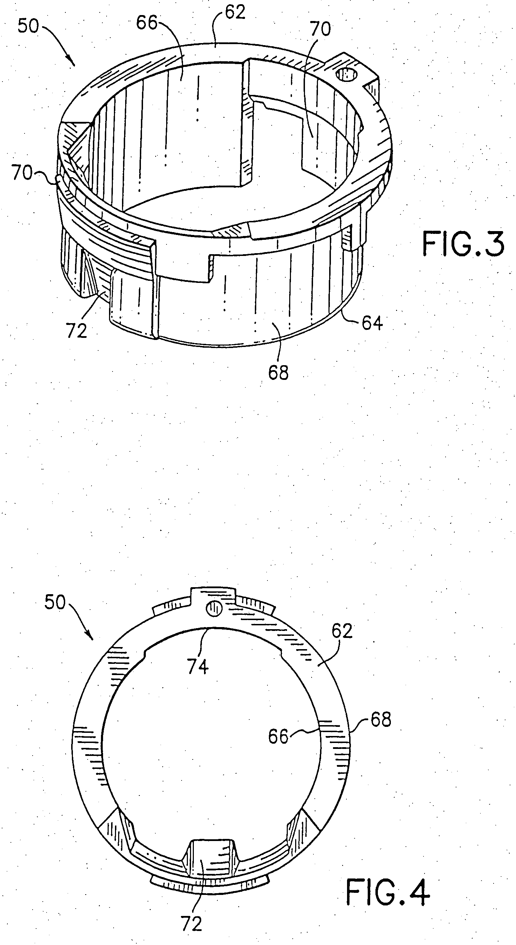

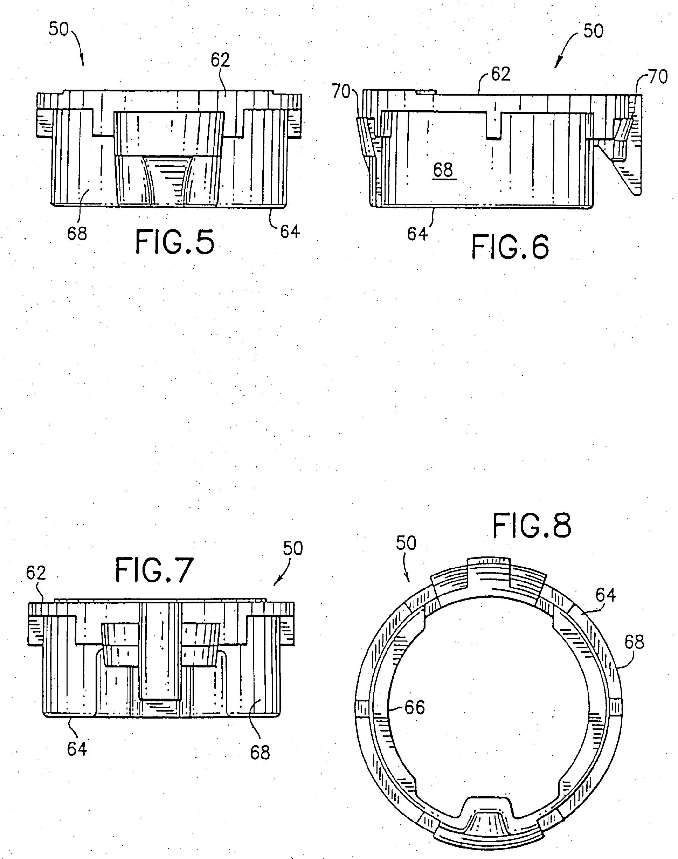

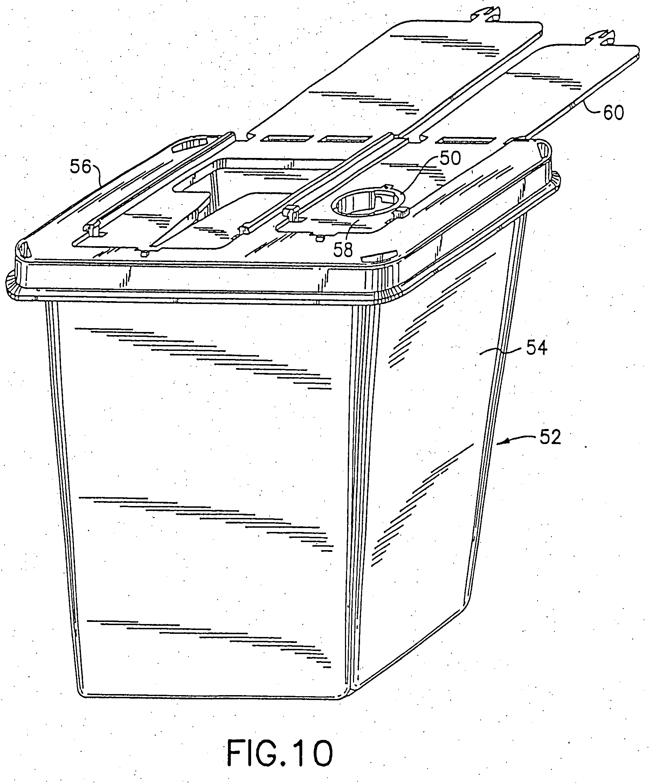

A needle disposal insert in accordance with the subject invention is identified by the numeral **50** in FIGS. 3-12 and is intended for use with needle holder **10** and needle assembly **12.** Needle disposal insert **50** further is usable with a sharps disposal container, such as sharps disposal container **52** illustrated in FIG. 10. Sharps disposal container **52** includes an open-topped receptacle **54** and a lid **56** snapped into secure engagement with the open top of receptacle **54**. Lid **56** is characterized by an opening **58** and a hinged cover **60**.

Needle disposal insert **50** is a generally tubular structure unitarily molded from a rigid plastic material and having a top end **62,** a bottom end **64,** an inner surface **66** and an outer surface **68**. Outer surface **68** of needle disposal insert **60** is configured for insertion into opening **58** in lid **56** of sharps disposal container **52.** Additionally, portions of outer surface **68** near top end **62** are characterized by opposed upwardly and downwardly facing lock surfaces **70** to permit snapped engagement of needle disposal insert **50** with portions of lid **56** adjacent opening **58.**

Most of inner surface **66** of needle disposal insert **50** is approximately cylindrical. However, inner surface **66** is characterized by a release section, identified generally by the numeral **72**, and a reset section, identified generally by the numeral **74**. Release section **72** is characterized by an upper slanted surface **76** that extends downwardly and inwardly from top end **62** of needle disposal insert **50** for approximately one third the length of needle disposal insert **50.** Release section **72** is characterized further by a vertical guide surface **78** aligned substantially parallel to the axis of tubular needle disposal insert **50** from the lower end of upper slanted surface **76** to a location approximately midway between top and bottom ends **62** and **64**. Release section **72** is characterized further by a lower slanted surface **80** that extends down from the lower end of vertical guide surface **78.** A lower vertical surface **81** extends from the bottom end of lower slanted surface **80.** Lower slanted surface **80** is aligned to the axis of needle disposal insert **50** at an angle of between 20° and 40°, and preferably about 28° and continues to bottom end **64** of needle disposal insert **50.**

Reset section **74** is characterized by an upper vertical surface **82** that extends down from top end **62** approximately one fourth the distance to bottom end **64**. The reset section is further characterized by a sloped reset surface **84** that extends down and out from the bottom end of upper vertical surface **82.** Sloped reset surface **84** extends for a distance at least equal to approximately one half the distance between top and bottom ends **62** and **64**. Reset section **74** is characterized further by a lower vertical surface **86** that extends from the bottom end of sloped reset section **84** to the bottom end **64** of needle disposal insert **50.** Slopped reset surface **84** is aligned to the axis of needle disposal insert **50** at an angle of between about 20° and 40°, and preferably about 24°. Thus, surfaces **80** and **84** may be approximately parallel.

The effective diameter that is formed between vertical guide surface **78** of release section **72** and a downward extension of upper vertical surface **82** of reset section **74** is only slightly greater than the outside diameter "a" of portions of needle holder **10** near end wall **26.** It follows that the effective diameter that is formed between the downward extension of upper vertical surface **82** and the lower sloped surface **80** on release section **72** at locations closer to bottom end **64** of needle disposal insert **50** becomes continuously smaller than the outside diameter "a" of needle holder **10.**

The radial offset between upper and lower vertical surfaces **82** and **86** on reset section **74** is identified by dimension "d" which is slightly greater than the radial offset "b" between the outer surface of flange **42** on movable jaw **38** and the outer circumferential surface of tubular sidewall **22** on holder **10,** as shown in FIG. 2.

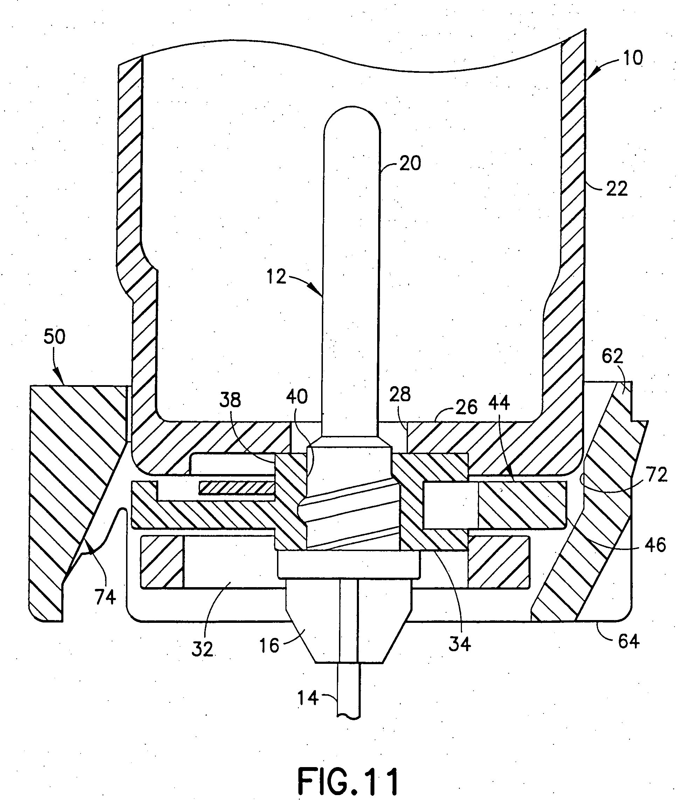

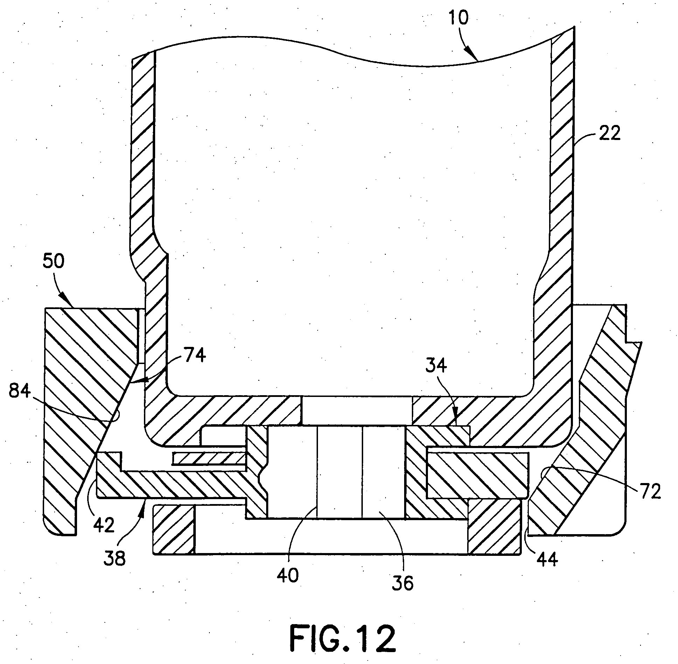

Needle disposal insert **50** is mounted in opening **58** in lid **56** of sharps disposal container **52** so that top end **62** of needle disposal insert **50** is substantially flush with or slightly above the top surface of lid **56.** Thus, bottom end **64** of needle disposal insert **50** projects into sharps disposal container **52.** Needle disposal insert **50** is employed as shown in FIGS. 11 and 12 by initially urging the assembly of needle holder **10** and used needle assembly **12** within the inner circumferential surface **62** of needle disposal insert **50.** Thus, the intravenous end of needle cannula **14** projects down through needle disposal insert **50**. Needle holder **10** is rotationally aligned so that first actuating surface **44** substantially registers with release section **72.** Needle holder **10** then is urged down so that outer circumferential surface regions of sidewall **22** slide against vertical guide surface **78** of release section **72** and upper vertical surface **82** of reset section **74**. Further downward movement of needle holder **10** urges actuating surface **46** of actuator **44** into sliding engagement with lower sloped surface **80** of release section **72.** However, the continued slidable engagement of sidewall **22** with vertical surfaces **78** and **82** prevents the entire needle holder **10** from translating. Thus, actuator **44** moves transversely relative to needle holder **10** and causes movable jaw **38** to translate transversely into the open position shown in FIG. 12. This translation of movable jaw **38** is permitted by the downwardly and outwardly sloped reset surface **84** of reset section **74**. As noted above, the offset "d" between upper and lower vertical surfaces **82** and **84** at opposite ends of sloped surface **84** is sufficient to permit the full range of translation of movable jaw **38**. Hence, used needle assembly **12** falls gravitationally into sharps receptacle **52.** The user may then pull needle holder **10** up and away from needle disposal insert **50**. The vertical surfaces **78** and **82** continue to hold tubular sidewall **22** of needle holder **10** in its original alignment. However, the outer surface of flange **42** on movable jaw **38** will slide against the upwardly and inwardly sloped surface **84** of reset section **74**. Hence, movable jaw **38** will be forced to translate radially inwardly and back into the closed position from FIG. 11. Thus, holder **10** is arranged to threadedly receive a new needle assembly **12** without any resetting by the user. Consequently, correct positioning of fixed jaw **34** and movable jaw **38** is assured by the mere separation of needle holder **10** from needle disposal insert **50.**

The illustrated embodiments relate to a needle disposal insert with rigidly fixed release and reset sections. However, it is within the scope of the invention to provide a needle disposal insert with release and/or reset sections that are spring driven, spring assisted or otherwise biased for movement in the direction of the slope to help move the movable jaw. Thus, less force will be required by the user.

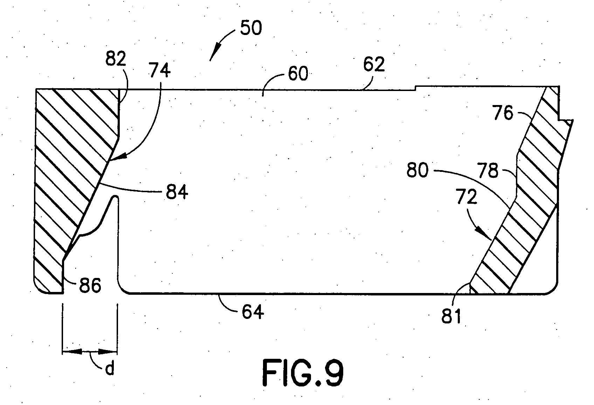

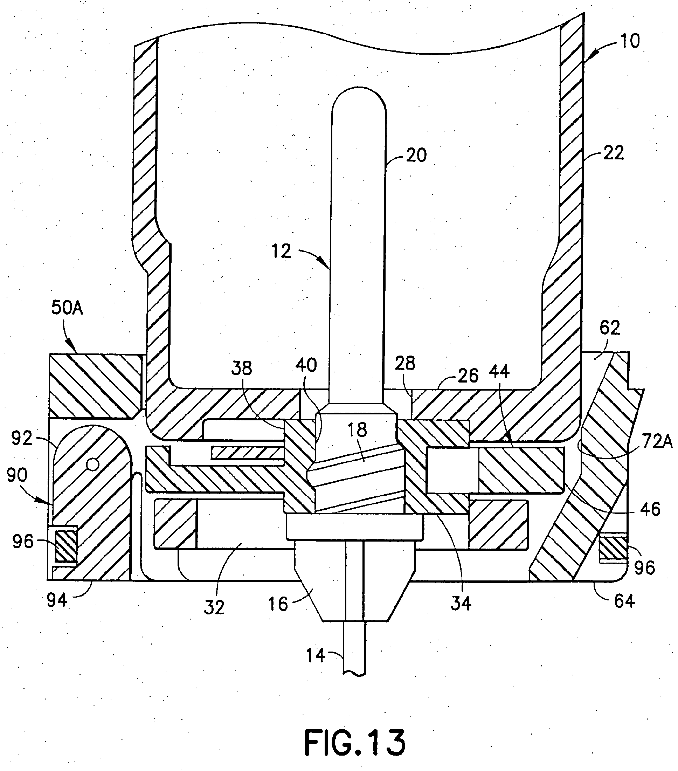

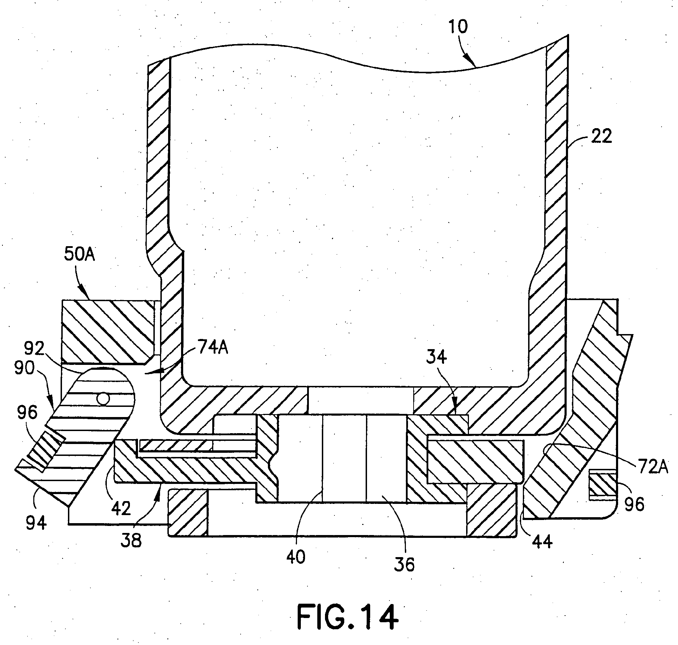

More particularly, an alternate needle disposal insert is illustrated in FIGS. 13 and 14 and is identified generally by the numeral **50A.** Needle disposal insert **50A** is used with a receptacle **52** identical to the receptacle **52** described above and illustrated in FIG. 10. Additionally, needle disposal insert **50A** is used with a needle holder **10** identical to needle holder **10** described and illustrated above. Insert **50A** is structurally and functionally very similar to insert **50** described and illustrated above. More particularly, insert **50A** includes a release section **72A** identical to the release section **72** described and illustrated with respect to the first embodiment of needle disposal insert **50.** However, needle disposal insert **50A** includes a reset section **74A** that is movable for actively assisting in the reset of needle gripping assembly **30**. In this regard, reset section **74A** includes a reset wall **90** with opposite top and bottom ends **92** and **94.** Top end **92** of reset wall **90** is connected pivotally to the remainder of needle disposal insert **50A.** Thus, bottom end **94** can pivot inwardly and outwardly relative to needle disposal insert **50A.** Needle disposal insert **50A** further includes biasing means **96** for urging reset wall **90** inwardly. Biasing means **96** is illustrated as being an elastic member that extends circumferentially around needle disposal insert **52**. However, other biasing means may be employed, such as a biasing means unitarily incorporated into the hinge connection or a radially aligned spring for exerting radially inward forces on reset wall **90**. Biasing means **96** normally urges reset wall **90** into a radially inner position as shown in FIG. 13. However, forces generated by the downward movement of needle holder **10** into needle disposal insert **50A** will cause lower end **94** of reset wall **90** to pivot out and against forces exerted by biasing means **96.** The outward pivoted alignment of reset wall **90** is similar to the alignment of sloped surface **84** on reset section **74** in needle disposal insert **50** of FIG. 9. Thus, reset wall **90** will perform in a similar manner to reset section **74** of needle disposal insert **50.** However, biasing means **96** will urge lower portions of reset wall **90** in a radially inward direction, thereby assisting in the reset of movable jaw **38.**

## Claims

1. A needle disposal insert having a substantially tubular sidewall with an upper end, a lower end and an inner circumferential surface extending between said ends, said inner circumferential surface being **characterized by** a release section and a reset section diametrically opposite said release section, said release section comprising an inwardly and downwardly sloped release actuating surface, said reset section comprising a downwardly and outwardly sloped reset actuating surface.

2. A needle disposal insert according to claim 1, wherein said release section further comprises a release guide surface extending substantially parallel to said tubular sidewall from said top end to said inwardly sloped release actuating surface.

3. A needle disposal insert according to claim 2, wherein said reset section further comprises a reset guide surface substantially parallel to said release guide surface and extending from said top end to said reset actuating surface.

4. A needle disposal insert according to any of claims 1-3, wherein said release actuating surface has upper and lower ends that are offset radially from one another by a selected release offset distance, and wherein the reset actuating surface has upper and lower ends that are offset radially from one another by a selected reset offset distance, said release offset distance and said reset offset distance being substantially equal.

5. A needle. disposal insert according to any of claims 2-4, wherein said release guide surface and said sloped reset actuating surface are substantially equally spaced from said top end.

6. A needle disposal according to any of claims 1-5, further comprising an outer surface having means for securely mounting said needle disposal insert to a planar wall.

7. A needle disposal insert according to any of claims 1-6, wherein said outwardly sloped reset actuating surface is pivotable relative to remaining portions of said needle disposal insert, said needle disposal insert further comprising biasing means for urging at least portions of said sloped reset actuating surface inwardly.

8. A needle disposal receptacle comprising a container having a lid, an opening formed through said lid for accessing interior portions of said container, a needle disposal insert according to any of claims 1-7.

9. A needle disposal system comprising:
a needle holder having a tubular sidewall, an end wall extending from said tubular sidewall and having a central opening therein, a release mechanism attached to said end wall, said release mechanism comprising a pair of jaws, at least one of said jaws being movable between a closed position where said jaws permit releasable attachment of a needle to said needle holder and an open position where said jaws release said needle, said movable jaw projecting transversely beyond said needle holder when said movable jaw is in said open position; and
a container having a lid, an opening formed through said lid for accessing interior portions of said container, a needle disposal insert according to any of claims 1-7 in said opening in said lid.

10. A needle system according to claim 9, wherein portions of said needle holder adjacent said bottom wall define an outside diameter, and wherein portions of said insert adjacent said top end are dimensioned for slidable insertion of said tubular sidewall of said needle holder.

11. A needle system according to claim 9 or 10, wherein said movable jaw has an outer surface substantially aligned with said tubular sidewall of said holder when said movable jaw is in said closed position.
